# EUROPEAN PATENT APPLICATION

(11) **EP 1 466 601 A2**
(43) Date of publication of application: **13.10.2004**
(21) Application number: 04004643.5
(22) Date of filing: 14.06.1999
(51) Int. Cl.: A61K 31/403, A61P 25/06

(54) **Prevention of migraine recurrence**

(30) Priority: 30.07.1998 GB 9816556
(62) Divisional of application: 99922459.5
(71) Applicant: Pfizer Limited, Sandwich, Kent CT13 9NJ (GB); PFIZER INC., New York, N.Y. 10017 (US)
(72) Inventor: Jackson, Neville Colin, Sandwich, Kent CT13 9NJ (GB); Uden, Stephen, Point Road Groton Connecticut 06340 (US)
(74) Representative: Motion, Keith Robert

(57) **Abstract**

The invention relates to a dual-, sustained-, controlled-, delayed- or pulsed-release pharmaceutical composition for the prevention of migraine recurrence comprising eletriptan, or a pharmaceutically acceptable salt thereof.

## Description

This invention relates to the use of eletriptan for the manufacture of a medicament for the prevention of migraine recurrence.

5HT_{1B/1D} receptor agonists, such as the compounds known as "triptans", have been shown to be highly effective for the treatment of migraine. Examples of such triptan derivatives include eletriptan, sumatriptan, naratriptan, rizatriptan, zolmitriptan, almotriptan and frovatriptan.

Eletriptan, 3-([1-methylpyrrolidin-2(R)-yl]methyl)-5-(2-phenylsulphonylethyl)-1H-indole, is disclosed in WO-A-92/06973. A preferred hydrobromide salt form of eletriptan is disclosed in WO-A-96/06842. WO-A-99/01135 discloses a pharmaceutical formulation comprising eletriptan hemisulphate and caffeine.

Migraine is generally classified into two types, "migraine with aura" and "migraine without aura". The aura is the complex of focal neurological symptoms which initiates or accompanies an attack.

Migraine with aura is commonly defined as an idiopathic, recurring disorder manifesting itself with attacks of neurological symptoms unequivocally localisable to the cerebral cortex or brain stem, usually gradually developing over 5-20 minutes and lasting for less than 60 minutes. Headache, nausea and/or photophobia usually follow neurological aura symptoms directly or after a symptom-free interval of less than one hour. The migraine headache usually lasts from 4 to 72 hours but may be completely absent.

Migraine without aura is commonly defined as an idiopathic, recurring headache disorder manifesting itself in attacks lasting from 4 to 72 hours. Typical characteristics of the migraine headache are unilateral location, pulsating quality, moderate or severe intensity, aggravation by routine physical activity and association with nausea, photophobia or phonophobia.

Most patients will exclusively have migraine attacks without aura. It also seems that patients that have frequent attacks with aura have attacks without aura as well. "Premonitory symptoms" may occur either hours or a day or two before a migraine attack (with or without aura). These symptoms frequently consist of general features such as hyperactivity, hypoactivity, depression, craving for special foods, repetitive yawning and similar atypical symptoms.

Migraine recurrence is classified as a separate condition from migraine itself and can be defined as the return of a moderate or severe migraine headache within 24 hours of the first dosing with medication, from a state of no or mild migraine headache within 2 hours of the first dosing with medication.

There is evidence that although a triptan derivative can provide effective relief of a migraine headache, the use of such a derivative actually results in the condition of migraine recurrence developing at a rate that is characteristic of the particular triptan derivative used. Indeed the typical migraine recurrence incidence rate per migraine attack is of the order of 30% when a triptan derivative is used.

A clear distinction must be drawn between either the treatment of migraine, that is to treat an established migraine headache, or the treatment of migraine recurrence, that is to treat an established migraine headache recurrence, and the prevention of migraine recurrence, that is treating a patient in anticipation of a migraine headache recurrence in order to prevent that recurrence. It should be noted that not all patients suffer from migraine recurrence as defined above.

To date, no 5HT_{1B/1D} receptor agonist has been shown to prevent migraine recurrence and this simply cannot be predicted for any particular compound, even if previously indicated for the treatment of migraine. Indeed, no "triptan" is currently indicated for the prevention of migraine recurrence. The reason for the prevention of migraine recurrence being unpredictable is that the aetiology of migraine recurrence is not understood. Further, little is known about the characteristics of patients that have a tendency to experience migraine recurrence or, alternatively, the characteristics of migraine headaches that are likely to recur.

Cephalagia, 14, 330-338 (1994), discloses that a 100mg oral dose of sumatriptan aborts about 60% of migraine attacks within 2 hours but that the headache may recur within 24 hours. If a second tablet of sumatriptan is administered after 2 hours this does not increase initial efficacy and neither prevents nor delays migraine recurrence. However, administration of a further tablet of sumatriptan is highly effective in treating the established migraine recurrence. Further, Neurology, 45, 1505-1509 (1995), discloses that migraine recurrence may occur within 24 hours in approximately 40% of migraine attacks successfully treated with a 6mg subcutaneous dose of sumatriptan. However, a 100mg oral dose of sumatriptan taken 4 hours after the initial 6mg subcutaneous dose does not prevent migraine recurrence, but it significantly delays the time to migraine recurrence.

It has now been surprisingly found that eletriptan can be used for the prevention of migraine recurrence.

Accordingly the present invention relates to the use of eletriptan, or of a pharmaceutically acceptable salt or composition thereof, for the manufacture of a medicament for the prevention of migraine recurrence.

Further, the present invention relates to a method for the prevention of migraine recurrence comprising administration to a patient of an effective amount of eletriptan, or a pharmaceutically acceptable salt or composition thereof.

The pharmaceutically acceptable salts of eletriptan include the acid addition and the base salts thereof.

Suitable acid addition salts are formed from acids which form non-toxic salts and examples are the hydrochloride, hydrobromide, hydroiodide, sulphate, bisulphate, nitrate, phosphate, hydrogen phosphate, acetate, maleate, fumarate, lactate, tartrate, citrate, gluconate, succinate, saccharate, benzoate, methanesulphonate, ethanesulphonate, benzenesulphonate, p-toluenesulphonate and pamoate salts.

Suitable base salts are formed from bases which form non-toxic salts and examples are the sodium, potassium, calcium, magnesium and zinc salts.

For a review on suitable salts see Berge et al, J. Pharm. Sci., 1977, 66, 1-19.

Preferred salts of eletriptan for use in the present invention are the hydrobromide and sulphate, including hemisulphate, salts.

Also included within the scope of the present invention are polymorphs, solvates and radiolabelled derivatives of eletriptan or a pharmaceutically acceptable salt thereof.

The pharmaceutically acceptable solvates of eletriptan and its pharmaceutically acceptable salts include the hydrates thereof.

A pharmaceutically acceptable salt of eletriptan may be readily prepared by mixing together solutions of eletriptan and the desired acid or base, as appropriate. The salt may precipitate from solution and be collected by filtration or may be recovered by evaporation of the solvent.

Eletriptan, or a salt thereof, can be administered alone but will generally be administered in admixture with a suitable pharmaceutical excipient, diluent or carrier selected with regard to the intended route of administration and standard pharmaceutical practice.

For example, eletriptan, or a salt thereof, an be administered orally or sublingually in the form of tablets, capsules, ovules, elixirs, solutions or suspensions, which may contain flavouring or colouring agents, for immediate-or controlled-release applications.

Such tablets may contain excipients such as microcrystalline cellulose, lactose, sodium citrate, calcium carbonate, dicalcium phosphate and glycine, disintegrants such as starch, croscarmellose sodium and certain complex silicates, and granulation binders such as polyvinylpyrrolidone, sucrose, gelatin and acacia. Additionally, lubricating agents such as magnesium stearate, glyceryl benhenate and talc may be included.

Solid compositions of a similar type may also be employed as fillers in gelatin capsules. Preferred excipients in this regard include lactose or milk sugar as well as high molecular weight polyethylene glycols. For aqueous suspensions and/or elixirs, eletriptan, or a salt thereof, may be combined with various sweetening or flavouring agents, colouring matter or dyes, with emulsifying and/or suspending agents and with diluents such as water, ethanol, propylene glycol and glycerin, and combinations thereof.

Eletriptan, or a salt thereof, can also be injected parenterally, for example, intravenously, intraperitoneally, intrathecally, intraventricularly, intrasternally, intracranially, intramuscularly or subcutaneously, or it may be administered by infusion techniques. It is best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

For oral and parenteral administration to human patients, the daily dosage level of eletriptan, or a salt thereof, will usually be from 0.1 to 4 mg/kg (in single or divided doses).

Thus tablets or capsules of eletriptan, or a salt thereof, may contain from 5 to 240 mg, preferably from 5 to 100 mg, of active compound for administration singly or two or more at a time, as appropriate. The physician in any event will determine the actual dosage which will be most suitable for an individual patient and it will vary with the age, weight and response of the particular patient. The above dosages are exemplary of the average case. There can, of course, be individual instances where higher or lower dosage ranges are merited and such are within the scope of this invention.

Eletriptan, or a salt thereof, can also be administered intranasally or by inhalation and is conveniently delivered in the form of a dry powder inhaler or an aerosol spray presentation from a pressurised container or a nebuliser with the use of a suitable propellant, e.g. dichlorodifluoromethane, trichlorofluoromethane, dichlorotetrafluoroethane, a hydrofluoroalkane such as 1,1,1,2-tetrafluoroethane (HFA 134A [trade mark] or 1,1,1,2,3,3,3-heptafluoropropane (HFA 227EA [trade mark]), carbon dioxide or other suitable gas. In the case of a pressurised aerosol, the dosage unit may be determined by providing a valve to deliver a metered amount. The pressurised container or nebuliser may contain a solution or suspension of the active compound, e.g. using a mixture of ethanol and the propellant as the solvent, which may additionally contain a lubricant, e.g. sorbitan trioleate. Capsules and cartridges (made, for example, from gelatin) for use in an inhaler or insufflator may be formulated to contain a powder mix of eletriptan, or a salt thereof, and a suitable powder base such as lactose or starch. Alternatively, eletriptan, or a salt therof, may be administered intranasally by delivery from a non-pressurised unit or multi-dose, pump-type device. Preferred formulations for intranasal administration include those comprising eletriptan, or a salt thereof, and caffeine or a cyclodextrin.

Alternatively, eletriptan, or a salt thereof, can be administered in the form of a suppository or pessary, or it may be applied topically in the form of a lotion, solution, cream, ointment or dusting powder. Eletriptan, or a salt thereof, may also be transdermally administered by the use of a skin patch.

For application topically to the skin, eletriptan, or a salt thereof, can be formulated as a suitable ointment containing the active compound suspended or dissolved in, for example, a mixture with one or more of the following: mineral oil, liquid petrolatum, white petrolatum, propylene glycol, polyoxyethylene polyoxypropylene compound, emulsifying wax and water. Alternatively, it can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

Preferred formulations of eletriptan, or a salt, thereof are disclosed in WO-A-92/06973, WO-A-96/06842 and WO-A-99/01135. Particulary preferred formulations of eletriptan, or a salt thereof, for use in the prevention of migraine recurrence include dual-, sustained-, controlled-, delayed- or pulsed-release formulations.

Sustained-release dosage forms are designed to release eletriptan to the gastro-intestinal tract of a patient over a sustained period of time following administration of the dosage form to the patient. Suitable dosage forms include:
(a) those in which eletriptan or a pharmaceutically acceptable salt thereof is embedded in a matrix from which it is released by diffusion or erosion,
(b) those in which eletriptan or a pharmaceutically acceptable salt thereof is present in or on a multiparticulate core which is coated with a rate controlling membrane,
(c) those in which eletriptan or a pharmaceutically acceptable salt thereof is present in a dosage form containing a coating impermeable to the drug where release is *via* a drilled aperture,
(d) those in which eletriptan or a pharmaceutically accepable salt thereof is released through a semi-permeable membrane, allowing the drug to diffuse across the membrane or through liquid filled pores within the membrane, and
(e) those in which eletriptan is present as an ion exchange complex that effectively functions as a controlled release "salt" form of the active compound (e.g. by use of a suitable anion exchange resin such as sodium polystyrene sulphonate).

The skilled person would appreciate that some of the above means of achieving sustained-release may be combined, for example, a matrix containing the active compound may be formed into a multiparticulate and/or coated with an impermeable coating provided with an aperture.

Pulsed-release formulations are designed to release the active compound in pulses over a sustained period of time following administration of the dosage form to the patient. The release may then be in the form of immediate- or sustained-release. Delay in release may be achieved by releasing the drug at particular points in the gastro-intestinal tract or by releasing drug after a pre-determined time. Pulsed-release formulations may be in the form of tablets or multi particulates or a combination of both. Suitable dosage forms include:
(a) osmotic potential triggered release forms (e.g. see US Patent no. 3,952,741),
(b) compression coated two layer tablets (e.g. see US Patent no. 5,464,633),
(c) capsules containing an erodible plug (e.g. see US Patent no. 5,474,784),
(d) sigmoidal releasing pellets (e.g. as referred to in US Patent no 5,112,621) and
(e) formulations coated with or containing pH dependent polymers including shellac, phthalate derivatives, polyacrylic acid derivatives and crotonic acid copolymers.

Dual-release formulations can combine the active compound in immediate-release form with additional active compound in sustained-release form. For example, a bilayer tablet can be formed with one layer containing eletriptan in an immediate-release form and the other layer containing eletriptan embedded in a matrix from which it is released by diffusion or erosion. Dual-release formulations can also combine the active compound in immediate-release form with additional active compound in pulsed-release form. For example, a capsule containing an erodible plug could liberate active compound initially and after a predetermined period of time further active compound may be delivered in immediate- or sustained-release form.

Preferred drug dual release profiles include
(a) immediate release followed by controlled release;
(b) immediate release followed by zero order release;
(c) immediate release followed by sigmodial release; and
(d) double pulse release.

Delayed-release formulations are designed to release the active compound a predetermined time after administration. The release from delayed-release formulations may be in the form of immediate-release or sustained-release.

Controlled-release formulations impart control with respect to the rate of release or the time of release, or both, of the active compound and include sustained-, pulsed-, dual- and delayed-release formulations.

It has now been surprisingly found that administration of a 5-HT_{1B/1D} receptor agonist, or a pharmaceutically acceptable salt thereof, in the form of a dual-, sustained-, delayed-, controlled- or pulsed-release formulation prevents migraine recurrence.

Further examples of 5-HT_{1B/1D} receptor agonists that may be used include sumatriptan, naratriptan, rizatriptan, zolmitriptan, almotriptan and frovatriptan.

The dual-, sustained-, delayed-, controlled- and pulsed-release formulations that can be used are as described above for eletriptan.

Accordingly the present invention further provides:
a) a dual-, sustained-, delayed-, controlled- or pulsed-release pharmaceutical composition for the prevention of migraine recurrence comprising a 5-HT_{1B/1D} receptor agonist, or a pharmaceutically acceptable salt thereof, and a pharmaceutically acceptable excipient, diluent or carrier;
b) the use of a 5-HT_{1B/1D} receptor agonist, or a pharmaceutically acceptable salt or composition thereof, for the manufacture of a dual-, sustained-, delayed-, controlled- or pulsed-release pharmaceutical composition for the prevention of migraine recurrence; and
c) a method for the prevention of migraine recurrence comprising administration to a patient of an effective amount of a dual-, sustained-, delayed-, controlled-or pulsed-release pharmaceutical composition comprising a 5-HT_{1B/1D} receptor agonist, or a pharmaceutically acceptable salt thereof.

### PHARMACOLOGICAL DATA

Patients experiencing an acute migraine attack were orally dosed with either 40 or 80 mg of eletriptan (in the form of a hydrobromide salt) as a tablet formulation. All of the patients that experienced migraine relief within a 2 hour period after the initial dosing had a either a second dose (of the same strength as that administered initially) of eletriptan (in the form of a hydrobromide salt) or placebo administered if either migraine recurrence occurred within 8 hours after initial dosing or, if no migraine recurrence occurred, as close to 8 hours after initial dosing as possible.

The above protocol was repeated if the patient experienced a second acute migraine attack at least 48 hours after the first attack.

The results obtained for migraine recurrence rates (RR) following the first and second migraine attacks are tabulated below.

**TABLE**

| Dosing Sequence | 40mg - placebo | 40mg - 40mg | 80mg - placebo | 80mg - 80mg |
|---|---|---|---|---|
| First attack RR % | 16.6 | 7.0 | 12.5 | 6.2 |
| Second attack RR % | 10.2 | 3.3 | 11.2 | 6.1 |

These tabulated data show that eletriptan prevents migraine recurrence since where a second dose of eletriptan was administered following successful treatment of the initial migraine headache, the number of patients experiencing a migraine headache recurrence was at least halved compared with placebo.

## Claims

1. A dual-, sustained-, controlled-, delayed- or pulsed-release pharmaceutical composition for the prevention of migraine recurrence comprising eletriptan, or a pharmaceutically acceptable salt thereof.

2. A dual-release composition as claimed in claim 1.

3. A sustained-release composition as claimed in claim 1.

4. A controlled-release composition as claimed in claim 1.

5. A delayed-release composition as claimed in claim 1.

6. A pulsed-release composition as claimed in claim 1.

7. A composition as claimed in claim 2 that will have an eletriptan, or a pharmaceutically acceptable salt thereof, release profile of immediate release followed by sigmoidal release.

8. A composition as claimed in claim 2 that will have an eletriptan, or a pharmaceutically acceptable salt thereof, release profile of immediate release followed by controlled release.

9. A composition as claimed in claim 2 that comprises eletriptan, or a pharmaceutically acceptable salt thereof, in immediate-release form with additional eletriptan, or a pharmaceutically acceptable salt thereof, in sustained-release form.

10. A composition as claimed in claim 2 that comprises eletriptan, or a pharmaceutically acceptable salt thereof, in immediate-release form with additional eletriptan, or a pharmaceutically acceptable salt thereof, in pulsed-release form.

11. A composition as claimed in claim 6 where the release is to be in the form of sustained-release.

12. A composition as claimed in claim 6 that is in the form of sigmoidal releasing pellets.

13. A composition as claimed in claim 3 in which eletriptan or a pharmaceutically acceptable salt thereof is present in or on a multiparticulate core which is coated with a rate controlling membrane.

14. A composition as claimed in claim 5 where the release is to be in the form of sustained-release.

15. A composition as claimed in any one of the preceding claims for oral administration.
